# EUROPÄISCHE PATENTSCHRIFT

(11) **EP 1 964 581 B1**
(45) Veröffentlichungstag und Bekanntmachung des Hinweises auf die Patenterteilung: **02.11.2011**
(21) Anmeldenummer: 07018993.1
(22) Anmeldetag: 27.09.2007
(51) Int. Cl.: A61L 17/14

(54) **Chirurgisches Nahtmaterial mit antimikrobieller Oberfläche und Verfahren zur antimikrobiellen Beschichtung chirurgischen Nahtmaterials**
Surgical sewing material with antimicrobial surface and method for antimicrobial coating of surgical sewing material
Matériel de suture chirurgical doté d'une surface antimicrobienne et procédé de revêtement antimicrobien de matériel de suture chirurgical

(30) Priorität: 25.10.2006 DE 102006051093
(43) Veröffentlichungstag der Anmeldung: 03.09.2008
(60) Teilanmeldung: 11002607.7
(73) Patentinhaber: Heraeus Kulzer GmbH, 63450 Hanau (DE)
(72) Erfinder: Vogt, Sebastian, Dr., 99092 Erfurt (DE); Büchner, Hubert, Dr., 64354 Reinheim (DE); Kühn, Klaus-Dieter, Dr., 35041 Marburg-Elnhausen (DE)
(74) Vertreter: Kühn, Hans-Christian

(56) Entgegenhaltungen:
- EP-A- 1 669 093
- WO-A-00/57933
- WO-A-02/069874
- WO-A-2005/072281
- GB-A- 1 090 421
- 19000101, 1. Januar 1900 (1900-01-01), XP009103496
- STORCH M L ET AL: "Experimental Efficacy Study of Coated VICRYL plus ANtibacterial Suture in Guinea Pigs CHallenged with Staphylococcus aureas" SURGICAL INFECTIONS, MARY ANN LIEBERT, LARCHMONT, NY, US, Bd. 5, Nr. 3, 1. Januar 2004 (2004-01-01), Seiten 281-288, XP009046727 ISSN: 1096-2964

## Beschreibung

Gegenstand der Erfindung sind chirurgisches Nahtmaterial mit antimikrobieller Beschichtung und Verfahren zur antimikrobiellen Beschichtung von chirurgischem Nahtmaterial.

Chirurgisches Nahtmaterial kann man hinsichtlich seines Aufbaus in monofile und in polyfile Fäden unterteilen. Polyfile Fäden können einen so genannten Dochteffekt zeigen. Das bedeutet, durch Kapillarwirkung wandert Gewebsflüssigkeit entlang des Fadens. Damit kann auch eine Migration von mikrobiellen Keimen verbunden sein, so dass sich entlang des Fadenmaterials Infektionen ausbreiten können. Es ist daher wünschenswert, chirurgisches Nahtmaterial so auszurüsten, dass eine Keimbesiedelung auf der Fadenoberfläche und damit eine Migration von Keimen entlang des Fadens wirksam verhindert wird.

Bei den resorbierbaren Nahtmaterialien sind polyfile, geflochtene Polyglykolid-Fäden von besonderer praktischer Bedeutung. Es gibt bisher auf dem Markt nur ein von der Firma Ethicon vertriebenes, auf Polyglykolid basierendes Fadenmaterial, das mit dem Antiseptikum Triclosan ausgerüstet ist. Bei diesem Antiseptikum handelt es sich um ein gechlortes Biphenylderivat, das eine antiseptische Wirkung auf Gram-positive Bakterien ausübt. Wünschenswert wäre jedoch ein Fadenmaterial mit einer wesentlich breiteren antimikrobiellen Wirksamkeit, bei der auch Gram-negative Bakterien, Hefen und Pilze mit erfasst werden. Auch bei der Gruppe der nichtresorbierbaren Fadenmaterialien sind polyfile Fäden üblich. So wird zum Beispiel polyfiler, chirurgischer Stahldraht in der Thoraxchirurgie eingesetzt. Es sind gegenwärtig keine kommerziell vertriebenen, antimikrobiell ausgerüsteten nichtresorbierbaren Nahtmaterialien bekannt.

Es sind eine Vielzahl von antiseptisch wirksamen Substanzen seit Jahrzehnten bekannt und im medizinischen Gebrauch. Neben in Wasser löslichen Chlorhexidinsalzen in der Zahnpflege sind vor allem Octenidindichlorid-Lösungen unter dem Namen Octenisept® von der Firma Schüle & Mayr in der Chirurgie zur Wunddesinfektion und zur Flächendesinfektion weit verbreitet. Beim Octenisept handelt es sich um wässrig-alkoholische Lösungen von Octenidindichlorid und Phenoxyethanol. Das Octenidindichlorid ((1,1'-(1,10-Decandiyl)bis[4-(octylamino)pyridinium]-dichlorid) zeichnet sich gegenüber anderen kationischen Antiseptika dadurch aus, dass es zweifach positiv geladen ist. Octenidindichlorid hat ein sehr weites Wirkungsspektrum und umfasst Gram-positive und Gram-negative Bakterien als auch Hefen und Pilze. Es wurde 1977 von der Sterling Inc. entwickelt.

Eine ähnliche Struktur besitzt Dequaliniumchlorid mit ebenfalls zwei kationischen Zentren. Dequaliniumchlorid (1,10-Decamethylenbis(4-amino-2-methyl-chinolinium)-dichlorid) wurde 1957 von Allen & Hanburys beschrieben.

Sowohl Octenidindichlorid als auch Dequaliniumchlorid zeichnen sich durch ein sehr breites Wirkungsspektrum und eine gute Verträglichkeit mit Weichgewebe, insbesondere mit Schleimhaut, aus. Entsprechend wurden z.B. Octenidindichlorid-haltige Mittel zur Wund und Schleimhautdesinfektion beschrieben (DE 101 09 925 A1). Octenidindichlorid und Dequaliniumchlorid zeigen allerdings keine ausgeprägte Haftwirkung auf der Oberfläche von üblichem chirurgischem Nahtmaterial.

GB 1 090 421 beschreibt ein chirurgisches Textilmaterial, das eine Beschichtung aufweist, die eine Fettsäure und Dequaliniumacetat enthält. Zur Beschichtung wird eine wässrige Emulsion verwendet.

Der Erfindung liegt die Aufgabe zu Grunde, ein chirurgisches Nahtmaterial zu entwickeln, das so ausgerüstet ist, dass es vor einer mikrobiellen Besiedelung geschützt ist und dass der antimikrobielle Schutz ein breites Keimspektrum erfasst. Weiterhin soll ein unkompliziertes Verfahren entwickelt werden, das eine antimikrobielle Beschichtung vom kommerziellen Nahtmaterial erlaubt. Das Verfahren soll so gestaltet sein, dass das Nahtmaterial nicht durch den Beschichtungsvorgang in seiner Struktur beeinträchtigt wird.

Überraschenderweise wurde gefunden, dass durch Zusatz mindestens einer Fettsäure Octenidindichlorid und auch Dequaliniumchlorid auf den Oberflächen von Nahtmaterial zur Haftung gebracht werden können. Die Antiseptika Octenidindichlorid und Dequaliniumchlorid können zusammen mit der/den als Haftvermittler wirkenden Fettsäure/n auf die Oberfläche von chirurgischem Nahtmaterial aufgebracht werden. Es ergibt sich, dass eine Beschichtung aus einem Gemisch aus mindestens einer Fettsäure und Octenidindichlorid und/oder Dequaliniumchlorid auf der Fadenoberfläche aufgebracht ist.

Als Fettsäuren bevorzugt werden Laurinsäure, Palmitinsäure und Stearinsäure. Die Fettsäuren müssen nicht unbedingt als reine Substanzen eingesetzt werden. Es ist auch möglich, mit Monofettsäureglyceriden, Difettsäureglyceriden und Trifettsäureglyceriden verunreinigte Fettsäuren einzusetzen. Weiterhin ist es möglich, Gemische aus Fettsäuren und oligomeren Milchsäure-estern zu verwenden.

Das Gewichtsverhältnis von Octenidindichlorid oder Dequaliniumchlorid zur Fettsäure beträgt vorzugsweise 1 zu 0,1 bis 1 zu 5 ist, besonders bevorzugt 1 zu 1.

Die Aufgabe der Erfindung wird auch durch ein Verfahren zur Beschichtung chirurgischen Nahtmaterials gelöst, bei dem das Material mit einer homogenen methanolischen Lösung von Octenidindichlorid und/oder Dequaliniumchlorid und mindestens einer Fettsäure benetzt wird und anschließend das Methanol verdampft wird, wobei sich eine Beschichtung auf der Fadenoberfläche ausbildet. Es ist auch möglich, anstelle von Methanol Gemische aus Methanol und anderen niederen Alkoholen, wie Ethanol, Propanol und Isopropanol, einzusetzen. Der Vorteil der Verwendung von methanolischen Lösungen besteht auch darin, dass übliche Polyglykolid-, Polydioxanon- und Poly-epsilon-caprolacton-co-L-lactid-Fäden durch kurzzeitige Einwirkung von Methanol nicht angequollen werden und damit die Fadenstruktur erhalten bleibt.

Die Erfindung wird durch die nachstehenden Beispiele näher erläutert, ohne jedoch die Erfindung zu beschränken.

### Beispiel 1:

In 28,800 g Methanol (Fluka) werden 300 mg Dequaliniumchlorid (Solmag) und 300 mg Palmitinsäure (Fluka) bei Raumtemperatur gelöst. Es entsteht eine klare Lösung. In diese Lösung wird ein 50 cm langes Stück eines geflochtenen Polyglycolid-Fadens (USP 2.0) getaucht. Anschließend lässt man das Methanol bei Raumtemperatur verdampfen. Die Masse der Beschichtung wird gravimetrisch erfasst. Die Masse der Beschichtung beträgt 0,6 mg.

### Beispiel 2:

In 28,800 g Methanol (Fluka) werden 600 mg Octenidindichlorid (Dishman Pharmaceuticals) und 600 mg Palmitinsäure (Fluka) bei Raumtemperatur gelöst. Es entsteht eine klare Lösung. In diese Lösung wird ein 50 cm langes Stück eines geflochtenen Polyglycolid-Fadens (USP 2.0) getaucht. Anschließend lässt man das Methanol bei Raumtemperatur verdampfen. Die Masse der Beschichtung wird gravimetrisch erfasst. Die Masse der Beschichtung beträgt 1,4 mg.

## Patentansprüche

1. Chirurgisches Nahtmaterial mit antimikrobieller Oberfläche, die eine Beschichtung aufweist, die (i) mindestens eine Fettsäure und (ii) Octenidindichlorid und/oder Dequaliniumchlorid enthält, wobei die Beschichtung erhältlich ist durch (i) Benetzung des Nahtmaterials mit einer homogenen methanolischen Lösung von Octenidindichlorid und/oder Dequaliniumchlorid und mindestens einer Fettsäure und (ii) anschließendes Verdampfen des Methanols.

2. Chirurgisches Nahtmaterial nach Anspruch 1, **dadurch gekennzeichnet, dass** die mindestens eine Fettsäure der Gruppe bestehend aus Laurinsäure, Palmitinsäure und Stearinsäure angehört.

3. Chirurgisches Nahtmaterial nach Anspruch 1, **dadurch gekennzeichnet, dass** das Gewichtsverhältnis von Octenidindichlorid oder Dequaliniumchlorid zur Fettsäure oder der Summe der Fettsäuren 1,0 zu 0,1 bis 1,0 zu 5,0 beträgt

4. Chirurgisches Nahtmaterial nach Anspruch 3, wobei das Gewichtsverhältnis 1,0 zu 1,0 beträgt.

5. Chirurgisches Nahtmaterial nach Anspruch 1, **dadurch gekennzeichnet, dass** die Beschichtung ferner oligomere Milchsäureester enthält.

6. Verfahren zur Beschichtung chirurgischen Nahtmaterials mit den Schritten
i) Benetzung des Fadenmaterials mit einer homogenen methanolischen Lösung von Octenidindichlorid und/oder Dequaliniumchlorid und mindestens einer Fettsäure,
ii) anschließendes Verdampfen des Methanols.

7. Verfahren nach Anspruch 6, wobei die methanolische Lösung von der Gruppe der Gemische aus Methanol und anderen niederen Alkoholen gebildet wird.

8. Verfahren nach Anspruch 7, wobei die Alkohole der Gruppe Ethanol, Propanol und Isopropanol angehören.

## Claims

1. Surgical suture material with antimicrobial surface that comprises a coating that contains (i) at least one fatty acid and (ii) octenidine dichloride and/or dequalinium chloride, whereby the coating can be obtained through (i) wetting the suture material with a homogeneous methanolic solution of octenidine dichloride and/or dequalinium chloride and at least one fatty acid and (ii) subsequent evaporation of the methanol.

2. Surgical suture material according to claim 1, **characterised in that** the at least one fatty acid belongs to the group consisting of lauric acid, palmitic acid, and stearic acid.

3. Surgical suture material according to claim 1, **characterised in that** the weight ratio of octenidine dichloride or dequalinium chloride and fatty acid or total fatty acids is 1.0 : 0.1 to 1.0 : 5.0.

4. Surgical suture material according to claim 3, whereby the weight ratio is 1.0 : 1.0.

5. Surgical suture material according to claim 1, **characterised in that** the coating further contains oligomeric lactic acid esters.

6. Method for coating surgical suture material comprising the steps of
i) wetting the suture material with a homogeneous methanolic solution of octenidine dichloride and/or dequalinium chloride and at least one fatty acid;
ii) subsequent evaporation of the methanol.

7. Method according to claim 6, whereby the methanolic solution is formed by the group consisting of mixtures of methanol and other lower alcohols.

8. Method according to claim 7, whereby the alcohols belong to the group consisting of ethanol, propanol, and isopropanol.

## Revendications

1. Matériau de suture chirurgical à surface antimicrobienne qui présente un revêtement qui contient (i) au moins un acide gras et (ii) du dichlorure d'octénidine et/ou du chlorure de déqualinium, dans lequel le revêtement peut être obtenu par (i) imprégnation du matériau de suture avec une solution méthanolique homogène de dichlorure d'octénidine et/ou de chlorure de déqualinium et d'au moins un acide gras et (ii) évaporation subséquente du méthanol.

2. Matériau de suture chirurgical selon la revendication 1, **caractérisé en ce que** l'au moins un acide gras appartient au groupe constitué de l'acide laurique, l'acide palmitique et l'acide stéarique.

3. Matériau de suture chirurgical selon la revendication 1, **caractérisé en ce que** le rapport pondéral entre dichlorure d'octénidine ou chlorure de déqualinium et l'acide gras ou la somme des acides gras est de 1,0 sur 0,1 à 1,0 sur 5,0.

4. Matériau de suture chirurgical selon la revendication 3, dans lequel le rapport pondéral est de 1,0 sur 1,0.

5. Matériau de suture chirurgical selon la revendication 1, **caractérisé en ce que** le revêtement contient en outre des esters d'acide lactique oligomères.

6. Procédé de revêtement de matériau de suture chirurgical comprenant les étapes de
i) imprégnation du matériau de fil avec une solution méthanolique homogène de dichlorure d'octénidine et/ou de chlorure de déqualinium et d'au moins un acide gras,
ii) évaporation subséquente du méthanol.

7. Procédé selon la revendication 6, dans lequel la solution méthanolique est formée par le groupe des mélanges de méthanol et d'autres alcools inférieurs.

8. Procédé selon la revendication 7, dans lequel les alcools appartiennent au groupe de l'éthanol, du propanol et de l'isopropanol.
